(19) Europäisches Patentamt
European Patent Office
Office européen des brevets

(11) Publication number: **0 095 932**
**B1**

(12) # EUROPEAN PATENT SPECIFICATION

(45) Date of publication of patent specification: **06.08.86**

(51) Int. Cl.⁴: **C 12 N 11/02**, C 12 N 11/08, C 08 F 20/22, C 08 L 33/16, C 07 K 17/02, G 01 N 33/53

(21) Application number: **83303121.4**

(22) Date of filing: **31.05.83**

(54) **The use of a particulate polymer as a carrier for biological substances and the like and such substances supported on the carrier.**

(30) Priority: **28.05.82 JP 89707/82**

(43) Date of publication of application:
**07.12.83 Bulletin 83/49**

(45) Publication of the grant of the patent:
**06.08.86 Bulletin 86/32**

(84) Designated Contracting States:
**DE FR GB**

(56) References cited:
**DE-A-2 552 510**
**DE-A-3 105 768**
**US-A-3 925 267**
**US-A-3 932 321**
**US-A-4 193 845**

**The file contains technical information submitted after the application was filed and not included in this specification**

(73) Proprietor: **JAPAN SYNTHETIC RUBBER CO., LTD.**
**11-24, Tsukiji-2-chome Chuo-ku**
**Tokyo 104 (JP)**

(72) Inventor: **Aotani, Seiji**
**6-502, Wakabadai-1-chome**
**Asahi-ku Yokohama (JP)**
Inventor: **Kanayama, Hisanori**
**23-8, Ogawa-2-chome**
**Machida-shi (JP)**

(74) Representative: **Tubby, David George et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 932 B1

**Description**

The present invention relates to the use of certain particulate polymers as carriers for supporting various materials, particularly biological substances, such as immunoreactive substances, enzymes, cells and cell-discriminating substances, most particularly immunoreactive substances.

In recent years, an important part of clinical examinations has involved the detection of immunoreactive substances (such as antigens or antibodies) by an immunological reaction with the complementary immunoreactive substance (such as an antibody or an antigen) supported on a particulate carrier. Moreover, discrimination between or separation of cells also often employs a particulate carrier on which is supported a substance capable of combining selectively with specific cells or with specific classes or cells, i.e. a cell-discriminating substance. Furthermore, it is conventional to conduct enzymatic reactions on a particulate carrier on which is supported the enzyme and to cultivate cells using a particulate carrier supporting the original cell culture as a cell-culture bed.

For example, the presence of an immunoreactive substance can be determined by mixing it with a dispersion (hereinafter referred to as the "sensitizing latex") in a liquid medium of an immunological reagent comprising a complementary immunoreactive substance (e.g. an antibody if the substance to be detected is an antigen, or an antigen if the substance to be detected is an antibody) supported on an appropriate carrier. Any change in the state of the sensitizing latex, e.g. aggregation, sedimentation or continued dispersion, is then observed.

By labelling the substance under investigation, as, for example, in enzyme immunoassay or radio immunoassay, it is possible to determine the amount of the substance which has reacted with the particles of the sensitizing latex and hence the amount and/or concentration in the test liquid. Furthermore, if an antigenic or antibody group is present in cells, these cells only can be selectively collected by using a carrier supporting an antibody or antigen corresponding to that antigenic or antibody group.

In these methods, it is desirable that the particulate carrier supporting the biological substances should be stable and essentially inert to the substances and/or reactions involved, i.e. it should not give a non-specific response, e.g. a non-specific reaction or non-specific aggregation. Moreover, it is desireable that the carrier should be easily separable from the liquid medium and that, when the carrier is reacted in the form of a sensitizing latex with the substance to be detected, any change in stage of that latex should be readily observable.

Hitherto, the materials used as such particulate carriers have been the erythrocytes of animals, such as humans, sheep, domestic fowl or alligators, or fine particles of certain specific types of polymer, such as polystyrene, styrene, styrene-acrylic acid copolymers or styrene-methacrylic acid copolymers.

Sensitizing latexes in which erythrocytes are used as the carrier have been used in such simple detecting methods as the slide method or the microtitre method, as changes in the state of the sensitizing latex can be observed relatively quickly after the sensitizing latex has been reacted with the substance under investigation. However, erythrocytes, and hence sensitizing latexes made from them, tend to vary greatly in quality and non-specific aggregation tends to occur; furthermore, it is difficult to store the erythrocytes and sensitizing latexes containing them. It would, therefore, be desirable to replace the erythrocytes by a more stable carrier. Accordingly, in some cases, fine particles of such polymers as polystyrene have been used as carriers to support biological substances, such as immunoreactive substances, either by physically adsorbing the substances, or by chemically combining with them. Such polymers have excellent stability and do not vary in quality. However, the use as a carrier of polymers such as polystyrene in sensitizing latexes for detecting the presence of various substances has the major disadvantage that very substantial periods of time are required to observe aggregation or sedimentation; in general, the period required will substantially exceed 10 hours and may even be as long as several tens of hours.

US patent specification No. 3,932,321 discloses a series of polymers of phenyl and phenoxyalkyl acrylates (in which the phenyl and acrylate moieties may be substituted or unsubstituted), which are useful as flame-retardant polymers. We have now discovered that polymers of this type, in particulate form, may be used as carriers for biological substances, which can be stable and of invariant quality and which permit easy observation of changes of state of sensitizing latexes containing them. The use of the polymers of the invention as carriers, accordingly, allows one to achieve the advantages of both erythrocytes and known polymers, without the disadvantages of either.

Accordingly, the present invention consists the use as a carrier for supporting a biological substance of a particulate polymer prepared by polymerizing a monomer of formula:

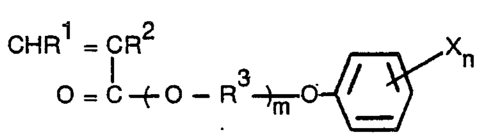

(I)

in which:

$R^1$ and $R^2$ are the same or different and each represents a hydrogen atom or an alkyl group having from 1 to 4 carbon atoms;

$R^3$ represents a straight or branched chain alkylene group having 2 or 3 carbon atoms and optionally having a hydroxy substituent;
X represents a halogen atom and, where there is more than one atom represented by X, the atoms may be the same or different;
*m* is 0 or an integer from 1 to 10; and
*n* is an integer from 1 to 5)
or by copolymerizing said monomer with at least at one other copolymerizable monomer, said monomer comprising at least 10 mole % of the total of all monomers.

Of the monomers having the above formula, we prefer those in which:
each of $R^1$ and $R^2$ represents a hydrogen atom or a methyl group;
$R^3$ represents an ethylene group or a propylene group;
X represents a chlorine atom or a bromine atom;
*m* is 0 or 1; and
*n* is an integer from 3 to 5.

Specific examples of such monomers include monofluorophenyl acrylate and methacrylate, monochlorophenyl acrylate and methacrylate, trichlorophenyl acrylate and methacrylate, pentachlorophenyl acrylate and methacrylate, monobromophenyl acrylate and methacrylate, tribromophenyl acrylate and methacrylate, pentabromophenyl acrylate and methacrylate, monoiodophenyl acrylate and methacrylate, 2-(tribromophenoxy)ethyl acrylate and methacrylate, 2-(tribromophenoxyethoxy)ethyl acrylate and methacrylate, 2-(pentachlorophenoxy)propyl acrylate and methacrylate and 2-hydroxy-1-(tribromophenoxy)propyl acrylate and methacrylate.

One of these monomers may be polymerized alone to form a homopolymer or two or more of the monomers of the above formula may be copolymerized to form a copolymer. Alternatively, by copolymerizing one or more of these monomers with one or more other copolymerizable monomers, the characteristics of the resulting carrier such as its hydrophilic or hydrophobic character, its functionality and the degree of charge, particularly at the surface of the carrier particles, can be varied and the specific gravity of the resulting polymer particles can be controlled.

Examples of suitable copolymerizable monomers include aromatic alkenyl compounds, alpha,beta-unsaturated carboxylic acids or their esters or amides, alpha,beta-unsaturated nitriles, halogenated vinyl compounds, conjugated dienes and lower fatty acid vinyl esters. Examples include styrene, chlorostyrene, alpha-methylstyrene, divinylbenzene, sodium styrenesulphonate, acrylic acid, methacrylic acid, methyl acrylate or methacrylate, ethyl acrylate or methacrylate, 2-hydroxyethyl acrylate or methacrylate, glycidyl acrylate or methacrylate, ethylene glycol diacrylate or dimethacrylate, allyl acrylate or methacrylate, acrylonitrile, methacrylonitrile, acrolein, methacrolein, acrylamide, methacrylamide, N-methylolacrylamide, N-methylolmethacrylamide, methylenebisacrylamide, methylenbismethacrylamide, butadiene, isoprene, vinyl acetate, vinylpyridine, N-vinylpyrrolidone, vinyl chloride, vinylidene chloride and vinyl bromide.

The polymer is preferably prepared by radical polymerization of the monomer, and various polymerization techniques may be employed, for example emulsion polymerization, soap-free polymerization (an emulsion polymerization employing no emulsifier), solution polymerization, solution-precipitation polymerization or seed polymerization, the soap-free polymerization, solution-precipitation polymerization and seed polymerization methods being preferred, since they allow the polymer to be formed as fine particles, hence allowing impurities to be removed easily.

By employing the soap-free polymerization method, and varying various factors, such as the way in which the monomer is added, the nature and amount of monomer and the amount of polymerization initiator, it is possible to obtain polymer particles having the same spherical shape and a particle diameter, which can be controlled to within the range from 0.05 to 2 micrometers with a narrow distribution of particle diameters. The medium used for soap-free polymerization may be water or a mixture of water with a water-soluble organic solvent, such as methanol, ethanol, acetone or tetrahydrofuran: these media are preferred in order to control the particle diameter and the polymerization rate.

In the case of the solution-precipitation polymerization method, polymer particles having a diameter within the range from 0.1 to 10 micrometers can be obtained by varying the nature and amount of solvent. In this case, the solvent used is one in which the initial monomer is soluble but in which the resulting polymer is insoluble. Examples include lower alcohols, ethers, ketones, esters, hydrocarbons and halogenated hydrocarbons, such as methanol, ethanol, isopropanol, anisole, dibutyl ether, acetone, methyl ethyl ketone, methyl isobutyl ketone, acetophenone, methyl acetate, ethyl acetate, butyl acetate, ethyl propionate, hexane, cyclohexane, benzene, dichloroethane, chloroform and chlorobenzene. However, the solubility of the monomer is not critical and it is sufficient that the monomer can be dissolved in the solvent by heating. Moreover, an ionic monomer, such as sodium styrenesulphonate, can sometimes be polymerized without dissolving it in a solvent. By using this polymerization method, when a polyfunctional monomer having a plurality of vinyl groups is copolymerized with the above-mentioned monomers, the diameter of the resulting polymer particles can easily be increased.

By using the seed polymerization method, for example by polymerizing monomer in the presence of fine particles (which may be polymer particles) by a soap-free polymerization method, or by suspension-polymerizing monomer adsorbed on and/or permeated into fine particles in a liquid medium such as water,

it is possible to increase further the particle diameter of the polymer particles and to narrow the particle diameter distribution.

The soap-free polymerization method is particularly preferred since the resulting polymer particles have a narrow distribution of particle diameters and have the same shape.

When a momomer having the aforementioned formula is polymerized with other copolymerizable monomers, it is preferred that the first-mentioned monomer should be present in an amount of at least 10 mole percent, more preferably from 15 to 60 mole percent; if the proportion of this monomer is too low, the ability of the resulting polymer particles to act as a carrier is markedly reduced and it is difficult to produce particles having an adequate specific gravity. In order to enable the polymer to be used most satisfactorily as a carrier in the present invention, the particle diameter is preferably within the range from 0.1 to 10 micrometers, more preferably from 0.3 to 1 micrometer, and the specific gravity is preferably from 1.2 to 2 g/cm$^3$, more preferably from 1.3 to 1.8 g/cm$^3$. If the particle diameter or specific gravity of the carrier is either too small or too large, it becomes difficult to observe any change in state of a sensitizing latex containing the carrier.

The means by which the biological substance, for example an immunoreactive substance, is supported on the carrier is not critical and any method commonly used may be employed. For example, the carrier may be contacted in a liquid medium with the immunoreactive substance and then separated and recovered, after which, if necessary, it may be washed with a detergent. The liquid medium chosen will vary, depending upon the kind of immunoreactive substance, although, in general, preferred media are a phosphate-buffer saline solution, a glycine buffer saline solution or a mixture of one or more of these with another material, such as a non-ionic surfactant or serum albumin. Furthermore, as the detergent, there may be usually used a liquid medium for use in supporting the immunoreactive substance on the carrier though it is not critical.

The carrier of the present invention, when used in the sensitizing latex, can make it easy to observe any change in state of the sensitizing latex caused by reaction of the carrier with the substance to be detected. Moreover, it can have excellent stability without any variation in quality and the surface characteristics of the carrier can easily be varied by appropriate selection of the monomers to be polymerized. In particular, the carrier of the present invention can support, per unit area, a relatively large quantity of biological or other materials and, moreover, it rarely undergoes non-specific aggregation and it has excellent dispersion stability, even when it is supporting a biological substance.

Since the carrier of the present invention will normally have a high specific gravity, it can be separated easily from liquid media, such as water, when used in the form of a sensitizing latex. Accordingly, it is particularly useful when sedimentation accompanies the detection of the substance under investigation. For example, when a reagent formed by supporting an immunoreactive substance on the carrier of this invention is used as the sensitizing latex in a microtitre method, the measurement, which has conventionally taken in excess of 10 hours and often several tens of hours, can be finished in about 30 minutes or, at most, several hours, and separation of the particles by centrifugation or other conventional means can also be carried out in a very short time.

Examples of biological substances which may be supported on the carrier of the present invention include, immunoreactive substances, such as the surface antigen of type B hepatitis (HBs antigen), anti-HBs antibody, human chorionic gonadotropin (HCG antigen), mycoplasma antigen, nucleic acid, nuclear protein, oestrogen and anti-oestrogen antibody; enzymes, such as glucose isomerase, glucose oxidase, alpha-amylase, papain or aminoacylase; and cells requiring a solid surface for their growth, such as foetal pneumocytes, renal cells and fibroblasts.

The invention is further illustrated by the following Examples:

Example 1

*Production of particles for carrier*

(1) A mixed solution of 64 g of pentachlorophenyl acrylate, 32 g of styrene, 3 g of methacrylic acid and 1 g of t-dodecylmercaptan was dropped continuously over a period of 4 hours, whilst stirring, into 600 ml of water containing 1 g of potassium persulphate and at a temperature of 80°C.

When all of the solution had been added, stirring was continued for a further 2 hours to enable the soap-free polymerization to go to completion. Polymer particles having an average diameter of 0.4 micrometer were obtained in the form of a latex at a polymerization conversion of 98%. These polymer particles were purified by washing, dispersed by supersonic treatment and then passed through pores having a diameter of 7 micrometers in order to remove aggregates of particles of a large diameter. Subsequently, the concentration of the residue was adjusted and the polymer particles, in the form of an aqueous dispersion, were used as a carrier.

The molar ratio of monomers used to produce this carrier was pentachlorophenyl acrylate: styrene: methacrylic acid = 0.37:0.55:0.8.

(2) 72 g of tribromophenyl acrylate, 26 g of styrene and 2 g of divinylbenzene were dissolved in 2 litres of ethanol, and then 2 g of azobisibutyronitrile were added. The mixture was then allowed to polymerise (precipitation polymerization) at 75°C with stirring, to deposit the polymer in the form of fine particles. Stirring was continued for 4 hours, giving particles having an average particle diameter of 1.0 micrometer in the form of a dispersion at a polymerization conversion of 93%. Since the polymer particles settled

immediately, they were separated from the ethanol by decanting, purified and then treated as in (1) above, after which they were used as a carrier in the form of an aqueous dispersion.

The molar ratio of the above-mentioned monomers was tribromophenyl acrylate: styrene: divinylbenzene = 0.41:0.55:0.03.

(3) To 300 g of a 10% by weight aqueous dispersion of polymer particles obtained as described in (1) above, were added 30 g of monomers in the same proportions as were used in (1) above and 0.6 g of benzoyl peroxide. The mixture was then stirred at room temperature for 2 hours, and then at 70°C for 5 hours, to effect polymerization (seed polymerization).

At the end of this time, the resulting polymer particles were purified and then treated as described in (1) above and used as a carrier in the form of an aqueous dispersion. The average particle diameter of this carrier was 0.55 micrometer and its particle diameter distribution was narrower than that of the carrier obtained as described in (1) above.

(4) A mixed solution of 30 g of 2-(tribromophenoxy)ethyl acrylate, 21 g of styrene and 2 g of methacrylic acid and a solution of 0.3 g of ammonium persulphate in 40 ml of water were continuously dropped from their respective vessels, with stirring, over a period of 3 hours into a medium consisting of 160 ml of water and 50 ml of acetone, which was maintained at 60°C. When the whole of the solutions had been added, stirring was continued for a further 2 hours to effect polymerizatiion (soap-free polymerization) and give polymer particles having an average diameter of 1.10 micrometers, in the form of a latex. These particles were purified and treated as described in (1) above, after which they were used in the form of an aqueous dispersion as a carrier.

Example 2

*Characteristics of carrier*

The characteristics of the various carriers obtained as described in Example 1, as well as, for comparison, the characteristics of carboxy-modified polystyrene particles having an average diameter of 0.5 micrometer were examined as follows.

(1) *Sedimentation time*

An aqueous dispersion containing the carrier in a concentration of 0.1% by weight was placed into a V-well of the type used for microtitration and the time required until a round spot could be observed clearly was measured.

(2) *Amount of protein adsorbed*

To 2 ml of an aqueous dispersion containing the carrier at a concentration of 0.5% by weight were added 2 ml of a solution of bovine serum gamma-globulin in a phosphate buffer which had a concentration of 0.5 mg/ml; they were then mixed for 1 hour. The gamma-globulin concentration in the supernatant was measured by means of a spectrophotometer at a wavelength 280 nm. The amount of gamma-globulin adsorbed per unit area of the carrier was calculated from the difference in absorbance between the sample and a blank.

(3) *Average particle diameter*

Measured by observation with an electron microscope.

(4) *Specific gravity*

Measured by a density-gradient tube method.

The results obtained are shown in Table 1.

TABLE 1

| Carrier | Average particle diameter (μm) | Specific gravity ($g/m^3$) | Sedimentation time | Amount of protein adsorbed ($\mu g/cm^3$) |
|---|---|---|---|---|
| Carrier obtained in Example 1—(1) | 0.40 | 1.56 | 360 min | 0.80 |
| Carrier obtained in Example 1—(2) | 1.00 | 1.69 | 110 min | 1.35 |
| Carrier obtained in Example 1—(3) | 0.55 | 1.56 | 240 min | 0.95 |
| Carrier obtained in Example 1—(4) | 1.10 | 1.45 | 160 min | 0.85 |
| Carboxy-modified polystyrene particles | 0.5 | 1.05 | > 48 hrs | 0.25 |

## Example 3

*Measurement of immunoreactive substances*

Each of the carriers obtained in Example 1—(1) and (2) was dispersed in a 1/60M phosphate buffer saline solution containing 0.15 mole of sodium chloride and having a pH of 7.2 (this buffer solution is hereinafter referred to as "PBS") in such an amount that the solids concentration was 0.25% by weight. To this dispersion was added the same volume of a PBS solution containing an antigen, thermally agregated human IgG (hereinafter referred to as "agr-IgG"), which had a concentration of 0.05 mg/ml. Each of the resulting mixtures was gently stirred for 2 hours in order to support the antigen on the carrier, after which it was washed with PBS to remove unadsorbed antigen and then diluted with a diluent prepared by adding rabbit serum to PBS to a concentration of 1% by weight (hereinafter referred to simply as "the diluent"), to prepare a sensitizing latex having a solids concentration of 0.25% by weight. The agr-IgG used here was an antigen obtained by dissolving human IgG in PBS to a concentration of 10 mg/ml and heating the resulting solution at a temperature of 63°C for 15 minutes.

For comparison, a sensitizing latex was prepared as described above, except that commercially available carboxy-modified polystyrene particles having an average diameter of 0.5 micrometer were used in place of the carriers produced as described in Example 1.

Following the microtitre method, serum from a rheumatic factor-positive patient was subjected to serial two-fold dilution with 0.025 ml of the diluent each time on a microtitration V-well. 0.025ml of the sensitizing latex described above was added dropwise to each dilution, after which they were mixed. The mixtures thus obtained were allowed to stand at room temperature and the aggregation image at each well bottom was assessed with the naked eye when judgement became possible. The results are shown in Table 2, from which it can be seen that, when the carriers of the present invention were used, the time required for the measurement was greatly reduced and the sensitivity was good.

TABLE 2

| Carrier | Dilution ratio | | | | | Judgment time |
|---|---|---|---|---|---|---|
| | 800 | 1600 | 3200 | 6400 | 12800 | |
| Carrier obtained in Example 1—(1) | +++ | +++ | ++ | ± | − | 240 min |
| Carrier obtained in Example 1—(2) | +++ | +++ | +++ | ++ | − | 60 min |
| Carboxy-modified polystyrene particles | +++ | +++ | + | − | − | 24 hrs |

Note: The symbols +++, ++, +, ± and − show the sedimentation states of particles: +++ shows the largest area and − shows the smallest spot, between ++, + and ± exist in this order.

Example 4

*Measurement of immunoreactive substance*

A PBS dispersion having a carrier concentration of 0.25% by weight was prepared from the carrier produced as described in Example 1—(3). With 1ml of this PBS dispersion was mixed 1ml of a solution (hereinafter referred to as "the diluted antigen") prepared by diluting human glomerular basement membrane antigen with PBS to 80 volumes. 1ml of a 0.125% by weight aqueous solution of chromium chloride was then added to the mixture, after which it was greatly stirred at 20°C for 2 hours, to allow the antigen to attach itself to the carrier. The mixture was then washed with PBS and the diluent, after which it was diluted with 1ml of the diluent to prepare a sensitizing latex. The antigen used here was the supernatent obtained by separating the glomerular basement membrane from the kidney of dead human, treating it overnight with collagenase at 37°C and then centrifuging it at 30,000 rpm for 60 minutes.

Meanwhile, 1ml of a PBS dispersion of formalin — anchored sheep erythrocytes having a concentration of 0.5% by weight was mixed with 1ml of a PBS solution of tannic acid having a concentration of 0.01mg/ml. The resulting mixture was allowed to stand at 37°C for 20 minutes, after which it was washed with PBS, giving a PBS dispersion of tannic acid-treated erythrocytes at a concentration of 0.5% by weight. 1ml of the diluted antigen was mixed with 1ml of this dispersion of tannic acid-treated erythrocytes and the resulting mixture was allowed to stand at 37°C for 40 minutes, to allow the antigen to attached to the erythrocytes. The mixture was then washed with PBS and the diluent, to give a sensitizing latex having a concentration of 0.5% by weight, for comparative purposes.

Using a microtitration method, an antibody prepared by immunising a rabbit with human glomerular basement membrane was subjected to serial 2-fold dilution, each time with 0.025ml of the diluent, on a microtitration V-type well', and 0.25ml of the appropriate sensitizing latex was added dropwise to each dilution, after which they were mixed thoroughly. The mixture thus obtained was allowed to stand at room temperature and the aggregation image at each well bottom was assessed by the naked eye at the time when judgement became possible. The results are shown in Table 3.

When the comparative sensitizing latex based on erythrocytes as the carrier was used, it was necessary, in order to prevent non-specific reactions, to add the same amount of the washed anchored sheep erythrocyte sediment to each of the solutions prepared by diluting the antibody, to treat each of the mixtures thus obtained at room temperature for 60 minutes, to centrifuge the mixture and finally to add the sensitizing latex dropwise to the resulting supernatent.

TABLE 3

| Carrier | Dilution ratio | | | Judgment time |
|---|---|---|---|---|
| | 16000 | 32000 | 64000 | |
| Carrier obtained in Example 1—(3) | + | ± | − | 3 hrs |
| Sheep erythrocyte | + | ± | − | 5 hrs |

Note: + ± and − are as defined in Table 2

It can be seen from Table 3 that the carrier of the invention, like sheep erythrocytes, can be used as a carrier for the antigen, but it requires a shorter judgement time than do sheep erythrocytes and it requires no pretreatment in order to prevent the occurrence of non-specific reactions.

## Example 5

*Measurement of immunoreactive substances*

The carrier obtained as described in Example 1—(4) was dispersed in PBS to a solids concentration of 0.5% by weight. Methylated bovine serum albumen was mixed with the dispersion to a concentration of 0.01 mg/ml, after which the mixture was allowed to stand for 2 hours and was then washed with PBS. An equal amount of a PBS solution of polyinosinic-polycytidylic acid (a double-stranded RNA) having a concentration of 0.1 mg/ml was then added and the resulting mixture was then gently stirred for two hours, to allow the antigen to attach itself to the carrier. The mixture was then washed with PBS, to remove the unadsorbed antigen, after which it was diluted with the diluent, to give a sensitizing latex having a solids concentration of 0.1% by weight.

By a microtitration method, the serum from a systemic lupus erythematosus positive patient was subjected to serial 2-fold dilution, each time with 0.025 ml of the diluent, in a microtitration V-well, and 0.025 ml of the sensitizing latex described above was added dropwise to each dilution and thoroughly mixed. The resulting mixtures were allowed to stand at room temperature and the aggregation image at each well bottom was assessed by the naked eye at the time when judgement became possible. The results obtained are shown in Table 4.

TABLE 4

| Carrier | Dilution ratio | | | Judgement time |
|---|---|---|---|---|
| | 160 | 120 | 640 | |
| Carrier obtained in Example 1—(4) | ++ | ± | − | 1 hr |

Note: ++, ± and − are as defined in Table 2.

**Claims**

1. The use as a carrier for supporting a biological substance of a particulate polymer prepared by polymerizing a monomer of formula:

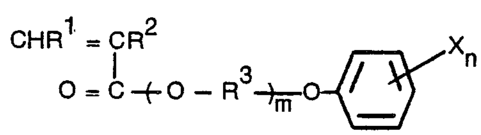

(I)

in which:

$R^1$ and $R^2$ are the same or different and each represents a hydrogen atom or an alkyl group having from 1 to 4 carbon atoms;

$R^3$ represents a straight or branched chain alkylene group having 2 or 3 carbon atoms and optionally having a hydroxy substituent;

X represents a halogen atom and, where there is more than one atom represented by X, the atoms may be the same or different:

*m* is 0 or an integer from 1 to 10: and

*n* is an integer from 1 to 5;

or by copolymerizing said monomer with at least at one other copolymerizable monomer, said monomer comprising at least 10 mole % of the total of all monomers.

2. The use of a carrier as claimed in Claim 1, in which the biological substance is an immunoreactive substance.

3. The use of a carrier as claimed in Claim 1 or Claim 2, said carrier having a particle diameter of from 0.1 to 10 micrometers.

4. The use of a carrier as claimed in any one of the preceding Claims, in which the carrier has a specific gravity of from 1.2 to 2 g/$cm^3$.

5. The use of a carrier as claimed in Claim 4, in which said specific gravity is from 1.3 to 1.8 g/$cm^3$.

6. The use of a carrier as claimed in any one of the preceding Claims, in which said other

copolymerizable monomer is an aromatic alkenyl compound, an alpha, beta-unsaturated carboxylic acid, an ester of an alpha, beta-unsaturated carboxylic acid, an alpha, beat-unsaturated carboxylic amide, an alpha, beta-unsaturated nitrile, a vinyl halide, a conjugated diene or a vinyl ester of a fatty acid.

7. The use of a carrier as claimed in any one of the preceding Claims, in which the monomer having said formula is monofluorophenyl acrylate or methyacyrlate, trichlorophenyl acrylate or methyacrylate, pentachlorophenyl acrylate or methacrylate, monobromophenyl acrylate or methacrylate, tribromophenyl acrylate or methacrylate, pentabromophenyl acrylate or methacrylate, monoiodophenyl acrylate or methacrylate, 2-(tribromophenoxy)ethyl acrylate or methacrylate, 2-(tribromophenoxyethoxy)ethyl acrylate or methacrylate, 2-(pentachlorophenoxy)propyl acrylate or methacrylate, or 2-hydroxy-1-(tribromophenoxy)propyl acrylate or methacrylate.

8. The use of carrier as claimed in any one of the preceding Claims, in which said partculate polymer is prepared by a soap-free polymerization method, a solution-precipitation polymerization method or a seed polymerization method.

9. A carrier comprising a particulate polymer, as defined in Claim 1, having supported thereon a biological substance.

10. A carrier as claimed in Claim 9, in which said biological substance is an immunoreactive substance.

11. A method of forming a product according to claim 9 or claim 10, comprising contacting the particulate polymer with the biological subtance in an aqueous medium.

12. A method according to claim 11, in which a dispersion of the particulate polymer in a buffered saline solution is contacted with an immunoreactive substance in solution in a similar medium.

**Patentansprüche**

1. Verwendung eines teilchenförmigen Polymers als Träger für biologische Substanzen, dadurch gekennzeichnet, daß das teilchenförmige Polymer durch Polymerisation eines Monomers der Formel

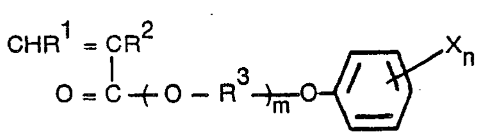

(I)

in der bedeuten:

$R^1$ und $R^2$, gleich oder verschieden, ein Wasserstoffatom oder eine $C_{1-4}$-Alkylgruppe,

$R^3$ eine geradkettige oder verzweigte, ggf mit einer Hydroxylgruppe substituierte $C_{2-3}$-Alkylengruppe,

X ein Halogenatom und, wenn mehr als ein X vorhanden ist, können diese gleich oder verschieden sein,

m 0 oder eine ganze Zahl von 1 bis 10 und

n eine ganze Zahl von 1 bis 5, oder

durch Copolymerisation dieses Monomers, in einer Menge von mindestens 10 mol-% der Gesamtmenge alier Monomere, mit mindestens einem anderen copolymerisierbaren Monomer, hergestellt wird.

2. Verwendung eines Trägers nach Anspruch 1, dadurch gekkenzeichnet, daß die biologische Substanz eine immunoreaktive Substanz ist.

3. Verwendung eines Trägers nach Anspruch 1 oder 2, dadurch gekennzeichet, daß er einen Teilchendurchmesser von 0,1 bis 10 µ hat.

4. Verwendung eines Trägers nach einem der vorhergehenden Ansprüche, dadurch gekennzeichnet, daß er ein spezifisches Gewicht von 1,2 bis 2 g/cm$^3$ hat.

5. Verwendung eines Trägers nach Anspruch 4, dadurch gekennzeichet, daß er ein spezifisches Gewicht von 1,3 bis 1,8 g/cm$^3$ hat.

6. Verwendung eines Trägers nach einem der vorhergehenden Asprüche, dadurch gekennzeichnet, daß als anderes co-polymerisierbares Monomer eine aromatische Alkenylverbindung, eine α,ß-ungesättigte Carbonsäure, ein Ester einer α,ß-ungesättigten Carbonsäure, ein α,ß-umgesättigtes Carbonsäureamid, ein α,ß-ungesättigtes Nitril, ein Vinylhalogenid, ein konjugiertes Dien oder ein Vinylester einer Fettsäure eingesetzt wird.

7. Verwendung eines Trägers nach einem der vorhergehenden Ansprüche, dadurch gekennzeichnet, daß als Monomer der Formel (I) Monofluorphenylacrylat oder -methacrylat, Trichlorphenylacrylat oder -methacrylat, Pentachlorphenyl- acrylat oder -methacrylat, Monobromphenylacrylat oder -methacrylat, Tribromphenylacrylat oder -methyacrylat, Pentabromphenylacrylat oder -methacrylat, Monojodphenylacrylat oder -methacrylat, 2-(Tribromphenoxy)ethylacrylat oder -methacrylat, 2-(Tribromphenoxyethoxy)ethylacrylat oder -methacrylat, 2-(Pentachlorphenoxy)propylacrylat oder -methacrylat oder 2-Hydroxy-1-(tribromphenoxy)propylacrylat oder -methacrylat eingesetzt wird.

8. Verwendung eines Trägers nach einem der vorhergehenden Ansprüche, dadurch gekennzeichnet, daß das teilchenförmige Polymer durch seifenfreie Polymerisation, Lösung -Fällungspolymerisation oder Keimpolymerisation hergestellt wird.

9. Träger aus einem teilchenförmigen Polymer gemäß Anspruch 1 mit daran gebundener biologischer Substanz.

10. Träger nach Anspruch 9, dadurch gekennzeichnet, daß die biologische Substanz eine immunoreaktive Substanz ist.

11. Verfahren zur Herstellung eines Produkts gemäß Anspruch 9 oder 10, dadurch gekennzeichnet, daß das teilchenförmige Polymer in einem wäßrigen Medium mit der biologischen Substanz in Berührung gebracht wird.

12. Verfahren nach Anspruch 11, dadurch gekennzeichnet, daß eine Dispersion des teilchenförmigen Polymers in einer gepufferten Salzlösung mit einer immunoreaktiven Substanz in einem gleichen Medium in Berührung gebracht wird.

**Revendications**

1. Utilisation, comme support d'une substance biologique, d'un polymére particulaire préparé en polymérisant un monomère de formule:

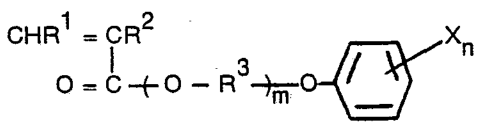

(I)

(dans laquelle:

$R^1$ et $R^2$ sont identiques ou différents et représentent chacun un atome d'hydrogène ou un groupe alkyl ayant 1 à 4 atomes de carbone;

$R^3$ représente un groupe alkylène à chaine droite ou ramifiée ayant 2 ou 3 atomes de carbone et comportant éventuellement un substituant hydroxyle;

X représente un atome d'halogène et, quand 11 existe plus d'une atome représenté par X, ces atomes peuvent étre identiques ou différents;

*m* est 0 ou un nombre entier de 1 à 10; et

*n* est un nombre entier de 1 à 5)

ou en copolymérisant ledit monomère avec au moins un autre monomère copolymérisable, ledit monomère constituant au moins 10 moles % du total de tous les monomères.

2. Utilisation d'un support sélon la revendication 1, dans laquelle la substance biologique est une substance immunoréactive.

3. Utilisation d'un support selon la revendication 1 ou la revendication 2, ce support ayant un diamètre particulaire de 0,1 à 10 micromètres.

4. Utilisation d'un support selon l'une quelconque des revendications précédentes, dans laquelle le support a une masse spécifique de 1,2 à 2 g/cm$^3$.

5. Utilisation d'un support selon la revendication 4, dans laquelle ladite masse spécifique est de 1,3 à 1,8 g/cm$^3$.

6. Utilisation d'une support selon l'une quelconque des revendications précédentes, dans laquelle ledit autre monomère copolymérisable est un composé alcénylique aromatique, un acide carboxylique, α,β-insaturé, un ester d'un acide carboxylique α,β-insaturé, un amide carboxylique β-insaturé, un nitrile α,β-insaturé, un hélogénure de vinyle, un diène conjugué ou un ester vinylique d'un acide gras.

7. Utilisation d'un support selon l'une quelconque des revendications précédentes, dans laquelle le monomère répondant à ladite formule est l'acrylate ou le méthacrylate de monofluorophényle, l'acrylate ou le méthacrylate de trichloropohényle, l'acrylate ou le méthacrylate de monobromophényle, l'acrylate ou le méthacrylate de tribromophényle, l'acrylate ou le méthyacrylate de pentabromophenyl, l'acrylate ou lea méthacrylate de monoiodophényle, l'acrylate ou le méthacrylate de 2-(tribromophénoxy)éthyle, l'acrylate ou le méthacrylate de 2-(tribromophénoxyéthoxy)éthyle, l'acrylate ou le méthacrylate de 2-(pentachlorophénoxy)propyle, ou l'acrylate ou le méthacrylate de 2-hydroxy-1-(tribromophénoxy)propyle.

8. Utilisation d'un support selon l'une quelconque des revendications précédentes, dans laquelle ledit polymère particulaire est préparé par un procédé de polymérization sans savon, un procédé de polymérisation par précipitation en solution ou un procédé de polymérisation sur germes.

9. Support constitué d'un polymère particulaire, tel que defini dans la revendication 1, portant une substance biologique.

10. Support selon la revendication 9, dans lequel ladite substance biologique est une substance immunoréactive.

11. Procédé de formation d'un produit selon la revendication 9 ou la revendication 10, consitant à mettre en contact le polymère particulaire avec la substance biologique dans un milieu aqueux.

12. Procédé selon la revendication 11, dans lequel une dispersion du polymére particulaire dans une solution saline tamponnée est mise en contact avec une substance immunoréactive en solution dans un milieu analogue.